# EUROPEAN PATENT SPECIFICATION

(11) **EP 3 235 464 B1**
(45) Date of publication and mention of the grant of the patent: **16.03.2022**
(21) Application number: 17166748.8
(22) Date of filing: 18.04.2017
(51) Int. Cl.: A61C 1/00, A61C 19/00, A61L 2/24

(54) **ANTI-STAGNATION DEVICE FOR A DENTAL TREATMENT UNIT**
STAGNATIONSSCHUTZVORRICHTUNG FÜR EINE ZAHNÄRZTLICHE BEHANDLUNGSEINHEIT
DISPOSITIF ANTI-STAGNATION POUR UNE UNITÉ DE TRAITEMENT DENTAIRE

(30) Priority: 21.04.2016 IT UA20162770
(43) Date of publication of application: 25.10.2017
(73) Proprietor: CEFLA Società Cooperativa, 40026 Imola (BO) (IT)
(72) Inventor: LENZI, Plinio, 40026 Imola (BO) (IT)
(74) Representative: Karaghiosoff, Giorgio Alessandro

(56) References cited:
- EP-A2- 0 734 692
- WO-A1-2008/018669
- US-A- 5 044 952
- US-B1- 7 056 472

## Description

The present invention relates to the technical field of dental treatment units for patients, also called dental units, used in dental practices. More particularly, the invention relates to an apparatus according to claim 1 and a method according to claim 7 to improve the hygienic quality of the water dispensed by dental instruments, using an anti-stagnation system in the water circuit of the dental unit itself.

Typically, dental treatments in human patients are performed on dental treatment units comprising a chair on which a patient lies, and a water unit supporting the instruments needed to provide dental therapies (e.g. dental syringe, micromotor, turbine, calculus scaler, etc.). An instrument or dentist table generally supports said instruments. Countless documents describe this well-known type of apparatus.

Typically, the above-quoted instruments are supplied with water, which has the aim of irrigating the area of treatment, preventing the overheating of the treated anatomic portion and allowing the cleansing of the treated area.

Said water usually comes from a water supply network; it passes through the dental unit: through a waterline is brought to instrument table, and from here the waterlines branch off to reach each of the already quoted dental instruments.

Mains water is not sterile, but should have a microbial count typical for potable water, i.e. in Europe should have an upper limit of 200 Colony Forming Units (CFU) per millilitre (CFU/ml). In fact, said water reaches patients' oral cavity, and it is possible that patients ingest a part of it. Moreover, according to the kind of dental therapy, it is possible that said water enters into contact with patient's blood flow. During the therapy, the dentist could reach a pulp chamber or interrupt the continuity of patient's oral mucosae. Therefore, irrigating water should have a limited microbial count, so as not to cause post-therapy infection in patients.

Nonetheless, it is well known that the passage of irrigating water through a dental unit leads to instruments distributing water of unsuitable quality in the oral cavity, i.e. with microbial counts in the order of some thousands of CFU/ml, even up to millions of CFU/ml (Guidelines for Infection Control in Dental Health-Care Settings, issued by US Centers for Disease Control in 2003). This can lead to serious health problems in patients, but even in dental staff, in that irrigating water, supplied as air/water spray, diffuses in the environment as a nebulization, and is inhaled by dental staff, who is therefore professionally exposed. This is even more worrying, in that some bacterial species frequently found in potable water are pathogen for humans, and have as their principal transmission modality airborne diffusion and inhalation.

The high contamination of the water distributed by dental units occurs in that the minimal bacterial count of the water entering into dental units, which as already said is not sterile but suitable for human consumption, finds in the waterlines of the dental unit itself conditions (like temperature, flow, and stagnation phases) very suitable for microbial colonization and multiplication, up to microbial concentrations much higher to that of the water entering into the dental unit.

Moreover, on the internal walls of the waterlines, from water entry into dental unit until the instrument distributing it into patient's oral cavity, progressively a biofilm forms, harbouring many different kinds of microorganisms, among which even pathogens for humans. The biofilm feeds microorganisms, allows them to multiply, and protects them from the environment, even from the disinfectants applied to disinfect waterlines. Water stagnation worsens significantly this phenomenon in waterlines during the non-use of water. Water stagnation also favours the depletion of the disinfectants introduced in mains water by water treatment plants or other local systems, and further favours biofilm colonization and growth.

To solve this problem many solutions were proposed, none of which is fully satisfying. E.g., there are dental treatment units wherein waterlines are periodically treated with chemicals, as described e.g. in EP1269844B1 Castellini.

This solution has some drawbacks, first of all the work required by dental assistant, who at the end of therapy (i.e. when patient's chair is empty) has to perform a disinfection cycle on the dental unit. Moreover, this solution requires downtimes of the patient's chair, in that when the dental unit is undergoing a disinfecting cycle, a patient cannot occupy it.

Always with the aim to improve the microbial quality of the water distributed by dental unit, other kind of solutions were proposed, like e.g. the introduction of anti-stagnation systems and procedures in dental units. Such systems have the aim to supply with a predetermined periodicity a given quantity of water, so as to prevent or reduce the deposit of biofilm on waterlines internal walls. The same above-quoted Guidelines recommend to flush waterlines, i.e. to release water from dental unit instruments for some minutes before starting to work, with the aim of replacing the liquid inside the waterlines, which is presumably contaminated, with new liquid which, not having stagnated in the dental unit, should possess the normal bacterial count of incoming water.

A solution recurring to anti-stagnation is described e.g. in EP0368818B1 always of Castellini. This document describes an anti-stagnation system, wherein there is provided a valve connected at least between a terminal part of the internal waterlines and the hydraulic drain of the apparatus, said valve being automatically triggered in the open position when the dental unit is started and in the closed position when the dental unit is switched off. Said valve, at least when the apparatus is switched on, generates a steady d of water from the circuit towards drain, with the aim of renovating the water present inside waterlines and preventing its stagnation, and biofilm deposition and formation in important quantities.

Nonetheless, the solution described in EP0368818B1 is not free from drawbacks. In fact, EP0368818B1 proposes a valve that is always open, with a marked consumption of water; this entails a regulation of the flow rate of this valve at minimal values in order to reduce consumption, and not to prevent or reduce the needed water flow from instruments. This also reduces the desired anti-stagnation effect. Moreover, the discharged water underwent heating to bring it to patient body temperature. This fact, with the low discharging flow and the consequent laminar flow of the liquid, further favours bacterial growth, in contrast with the aim itself of the anti-stagnation system.

Document EP0734692 discloses a hygiene system applicable on a dental unit comprising anti-stagnation unit positioned on a branch, downstream of the instruments so as to obtain a controlled and continuous release of water. The anti-stagnation unit is activated by hand by means of a control unit.

Document US5044952 discloses a device to prevent water stagnation in dental supply circuits. The device consists in a variable restriction installed in conjunction with a chip blower, located between the warm water inlet and the return line connecting the relative handpiece to the main waste outlet, which is made to open continuously or intermittently, at least while the apparatus is operating, so as to allow a moderate trickle of water out to the waste and ensure that no stagnation occurs.

Document US7056472 discloses a device for feeding a treatment liquid to medical appliances. The device includes an outlet opening for the treatment liquid, especially for feeding water into dental equipment, a feed line for the treatment liquid, means to introduce degerminating agents into the treatment liquid, and an on/off valve disposed upstream from an outlet opening onto which the feed line is connected. A back-flush valve is connected to the on/off valve, and a reservoir is provided for the treatment liquid, whereby the treatment liquid is transported from the reservoir with the aid of a pump. Further, a back-flush line is connectible alternatively via a drain valve to a drain or to the reservoir so that the treatment liquid is pumped into the drain, or in circulation from the reservoir through the feed line to the switch valve and through the back-flush line again into the reservoir.

Document WO2008018669 discloses a dental water processing system which senses the pressure or the flow rate of dental water in a pipe at regular intervals, and, when the sensing result corresponds to the operating condition inputted by a user or a manufacturer, automatically circulates the dental water in the pipe, thereby preventing a biofilm, etc. from forming on the inner surface of the pipe, or which necessarily circulates or purges dental water in a pipe for a predetermined period at a predetermined time, thereby preventing a biofilm, etc. from forming on the inner surface of the pipe. The device according to this document operates by circulating the water by means of a circulation pump between the dental unit water pipes and a water purifier. The water treatment circulation is triggered by a pressure or a flux sensor.

US5044952 also discloses a device designed to prevent the stagnation of water in the supply pipelines of medical equipment, and in particular, of dental surgery apparatus.

Aim of the present invention is providing an apparatus and a method allowing to perform an effective renewal of water in waterlines inside dental units, so as to prevent stagnation and reach a better microbial quality of the water distributed by the dental unit to patient's oral cavity.

This object is achieved by an apparatus and a method having the features of the independent claim. Advantageous embodiment and refinements are specified in the claims dependent thereon.

Substantially the present invention consists in inserting an active valve, which opens or closes or regulates a discharge flow, placed along the internal circuit of water distribution to the sundry consumption points, preferably placed in the instrument table of the dental unit, in the terminal point of the waterlines bringing water to the single dental instruments. The discharge flow, according to the functioning program, can be excluded during water distribution by instruments, and therefore does not jeopardize the correct distribution of spray. The programming of opening (on-off) allows also to regulate and reduce water consumption to the minimum.

The opening can be programmed by duration and time intervals of activation, so as to generate a discharge flow in suitable quantities, for a suitable duration, at suitable time intervals to prevent stagnation, i.e. when water consumption is inactive or for a inactivity time pre-determinable by the program, or automatically when dental unit is started at the beginning of a working day, as a step of the automatic procedure of dental unit starting.

An analogous device with an active valve can be applied to the assistant table, too, where instruments distributing water to patients are installed (e.g. a dental syringe or a calculus scaler), with analogous functioning of opening a discharge path, with activation possible both at the same time with the activation of the active valve in instrument table, or in sequence with it.

The opening of said valve or valves can also be manual, with a suitable command usable by dental staff, and the possibility to set time and modality of opening, in addition and independently from the activation program.

The activation of the valve/s can trigger the simultaneous or sequential activation of the water-to-cup spout, therefore performing the same anti-stagnation action on the water supply line of the water for dental rinsing, too.

A first advantage of the present invention is its quick effect, i.e. the renewal speed of the liquid present in the circuit. This is because the opening of the discharge is programmable and not continuous, and therefore allows to use significant flow rates when actioned. This without the limitations of flow rate that would be needed with the simultaneous opening of dental units points of consumption, not to interfere with the normal distribution of water by instruments.

A second advantage of the present invention consists in the enhancement of efficacy of other disinfecting systems present in the dental unit or even external to it, e.g. on the water supply line of the dental unit. The rapid and effective change of liquid in the circuit performed by the anti-stagnation device prevents the depletion of disinfectants in it and recalls in the circuit new supplies of water, containing fresh disinfectant, more effective and with optimal concentrations of active ingredient. The same phenomenon occurs even in the potable water mains upstream the dental unit. It shares with the dental unit both the low flow rate, with consequent laminar flow favouring biofilm colonization, and stagnation phases, with the consequence of increasing water temperature to the values of room temperature, favouring a higher microbial proliferation. In the absence of an anti-stagnation system, even on this waterline there is a progressive degradation of the disinfectant inserted by water treatment plants, up to its full depletion.

A third advantage of the present invention is the versatility of application, thanks to the programmability of working of the active valve. This allows to program the intervention of the anti-stagnation system according to pre-determined time intervals or in correspondence to defined states of use of the dental unit, e.g. after a programmed time of non-use of water, and also to regulate its intensity of action by programming times and fixed or intermittent modalities of opening.

Further advantages and properties of the present invention are disclosed in the following description, in which exemplary embodiments of the present invention are explained in detail based on the drawings:
- Figure 1: Axonometric view of a dental treatment unit according to the known art;
- Figure 2: Schematic representation of the water circuit according to the present invention in a dental unit without an assistant table;
- Figure 3: Schematic representation of the water circuit according to the present invention in a dental unit with an assistant table.

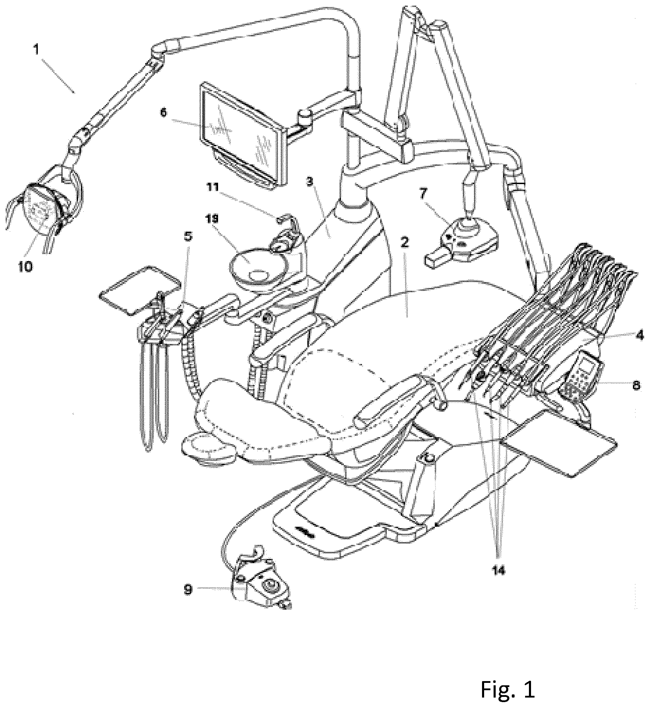

Figure 1 shows a dental treatment unit 1 according to the known art, comprising a patient chair 2 and a water unit 3. The water unit 3 typically supports a cuspidor 19, an instrument or dentist table 4 provided with a keyboard 8 for inputting commands and with a plurality of instruments 14 distributing water (dental syringe, micromotor, turbine, calculus scaler, etc.); an assistant's table 5, an operating lamp 10. The water group 3 supports also a water-to-cup spout for the water filling of a patient's cup (not shown). The unit 1 is provided with a foot control 9 and can optionally comprise a screen 6 and an intra-oral radiographic apparatus 7.

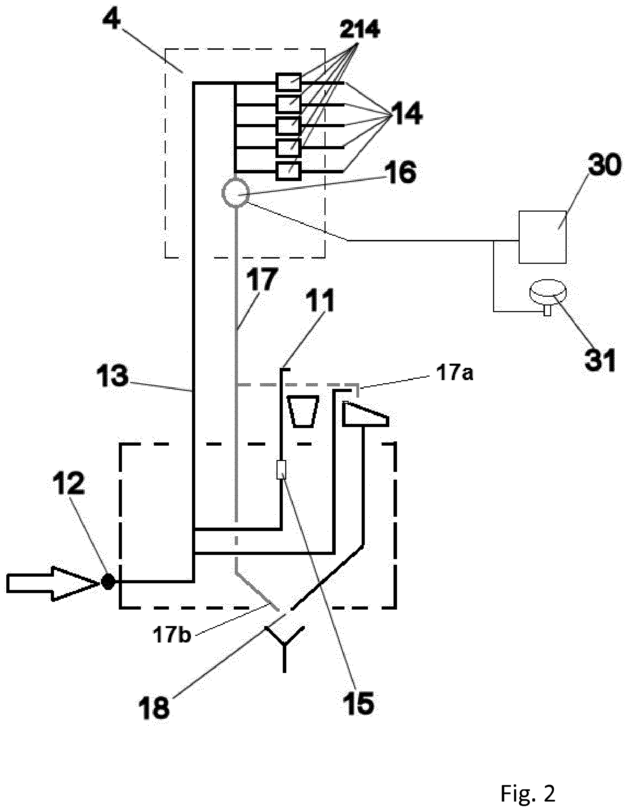

Figure 2 shows the entry point 12 of potable water into dental unit 1, an internal circuit 13 for distributing potable water to a plurality of instruments 14 supported on the instrument table 4 and to water-to-cup spout 11. Each instrument 14 is provided with an own valve 214 for the supply of the water, placed inside the instrument table 4. Figure 2 also shows an anti-stagnation valve 16, a line 17 of anti-stagnation discharge leading to dental unit drain 18 of dental unit.

It is worth noting that water can be discharged out of the dental unit in two alternative ways:
- Using the line 17b towards the drain 18 of dental unit;
- Using the line 17a of the cuspidor towards the drain 18 of dental unit.

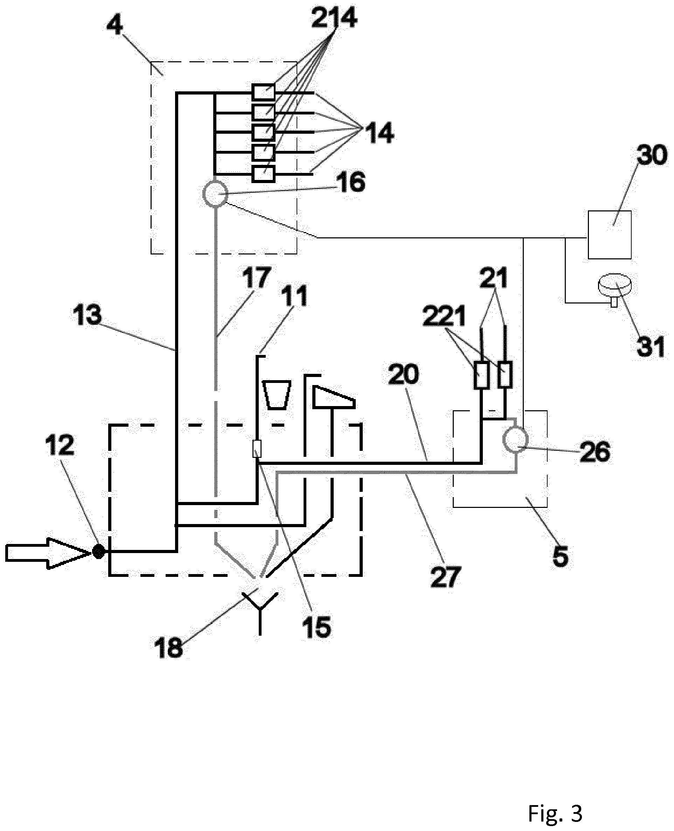

Figure 3 shows a further embodiment comprising the same components of Figure 2, with the addition of the assistant table 5, and the corresponding circuit portions. Figure 3 also shows a water circuit branch 20 distributing potable water to assistant table 5, branching off from circuit 13; instruments 21 supported by the assistant table 5, an anti-stagnation valve 26, a discharge line 27 for the assistant table discharging water in the drain 18 of dental unit 1. Each instrument 21 capable of distributing water is provided with its own valve 221.

In this case, too, water can be discharged in two alternative ways:
- Using the line 17b towards the drain 18 of dental unit;
- Using the line 17a of the cuspidor towards the drain 18 of dental unit.

The embodiment discharging water through the line 17a towards the cuspidor is particularly advantageous, in that the anti-stagnation discharge can be exploited to rinse the cuspidor, the air gap of the cuspidor can be exploited to separate discharge and water mains, and moreover dental staff (dentist, assistant) can visually verify the discharge of water performed by program.

In both the embodiments of the circuit there are provided an automatic program 30 and/or a manual control 31 for activating the anti-stagnation system.

In the normal use of the dental unit for treating a (not shown) patient, the dental unit 1 receives potable water from the connecting point 12; said water supplies the internal circuit 13 towards the instruments 14 supported by instrument table 4, the instruments 21 supported by assistant table 5 and water-to-cup spout 11.

In a point of the circuit 13, supplying instruments 14, preferably at an end point, a valve 16 for activating anti-stagnation system connects the circuit 13 with an anti-stagnation discharge line 17 merging into the drain 18 of dental unit.

In correspondence with programmed steps of use or non-use of instruments making use of water, a program 30 activates the valve 16 causing a flow in the discharge line 17 up to the general discharge line of the dental unit. This collects all the liquids in the water circuit 13, and therefore causes the entry into the same water circuit 13 of new potable water from the connecting point 12. In fact, the water circulating in the dental unit is under pressure; therefore, the opening of the anti-stagnation discharge circuit provokes the entry of new water from the supply point.

Optionally, the opening of the anti-stagnation activation means 16 at the same time or sequentially generates the opening of water-to-cup spout 11 supply means, realizing the renewal of water also in that branch of circuit 13.

Optionally, the anti-stagnation system can involve also the branch 20 of the water circuit directed to the assistant table 5, and ending with means 26 of anti-stagnation activation connected to a discharge anti-stagnation line 27 from assistant table 5 directed to the general drain 18 of the dental unit.

Said valve can be realized either as independent device, or as an internal component of an already existing valve for supplying liquid to one of the instruments 14, realizing it as a three-way valve, one of which connects the water circuit of the dental unit to the anti-stagnation discharge line in an alternative way to the normal connection with the instrument for supplying liquid. In an alternative embodiment, said third way can be placed on any valve 214 or 221 of an instrument supplying water, both in instrument table 4, and assistant table 5.

The activation of anti-stagnation devices 16 and optionally 26 can occur through an automatic program 30, according to a calculation of the time elapsed since the last use of water. E.g., if the dentist is performing a therapy on a patient using a turbine, the program starts its time calculation from the end of turbine use up to a pre-set interval of time, at the reaching of which the program will activate the anti-stagnation valve(s).

Alternatively, the program 30 can calculate the non-use time of the dental unit: e.g. during the midday break, the anti-stagnation system can activate at regular programmed intervals.

In a preferred embodiment, the anti-stagnation system activates at the switching on of the dental unit, e.g. at the beginning of the working day, realizing automatically the recommendations of the US CDC of discharging and replacing the water in the dental water through a flushing procedure to be performed at the beginning of each working day.

If the dental unit 1 is provided with disinfection systems of the water circuit 13, the program 30 activates the anti-stagnation systems 16 and optionally 26 even in correspondence of suitable steps of the disinfecting cycle, without impeding the normal working of the process, and introduces the same disinfectant also in the anti-stagnation lines 17 and 27, in addition to discharging the disinfectant from all the instruments involved in the disinfection cycle.

In a further embodiment, the water circuit 13 is provided with commercial means, known to the skilled man, to evaluate the bacterial count present in the water circulating inside the dental water. When a limit value is crossed, the program 30 activates the anti-stagnation system.

Obviously, the user always has at her/his disposal the manual command 31, allowing to perform an anti-stagnation cycle in any moment.

The skilled man knows the data and has the capabilities to calculate the volume of water contained in a circuit filled of water; moreover knows the flow rate of the anti-stagnation valve. It is apparent that the opening times of the valve have to be determined so as to guarantee, as a minimum, the discharge of a quantity of stagnating water equal to or higher than one volume of water contained in the circuit.

This system guarantees that the water in the circuit 13 is always of recent supply and consequently at its maximum of anti-bacterial protection.
- 1: dental treatment unit or dental unit
- 2: chair
- 3: water group
- 4: instrument or dentist table
- 5: assistant table
- 6: screen
- 7: intra-oral radiographic apparatus
- 8: keyboard
- 9: foot control
- 10: operating lamp
- 11: water-to-cup spout
- 12: entry of potable water
- 13: water circuit
- 14: instruments supported by intstrument table
- 15: activation of water to cup spout
- 16: anti-stagnation valve
- 17a: anti-stagnation discharge line to cuspidor
- 17b: anti-stagnation discharge line to general discharge
- 18: drain
- 19: cuspidor
- 20: potable water circuit to assistant table
- 21: instruments supported by assistant table
- 26: anti-stagnation valve
- 27: anti-stagnation discharge line to assistant table
- 30: automatic program
- 31: manual control
- 214: valves to instruments 14
- 221: valves to instruments 21

## Claims

1. Dental unit (1) comprising a water circuit (13) supplying potable water from entry point (12) to instruments (14) supported by instrument table (4), comprising an anti-stagnation system wherein
said anti-stagnation system comprises a valve (16) placed along the water circuit (13) supplying instruments (14) on instrument table (4); said valve being open or closed, according to an automatic program (30) and/or a manual control (31); when open, it directs water from circuit (13) to an anti-stagnation discharge line (17a or 17b) leading to dental unit drain (18),
the said anti-stagnation system being provided with the automatic program (30), the manual control (31) and J two alternative discharge lines consisting in:
- a line (17b) towards the drain (18) of dental unit and
- a line (17a) of the cuspidor towards the drain (18) of dental unit,
- said automatic program (30) being configured to determine programmable opening times of the anti-stagnation valve (16) to guarantee, as a minimum, the discharge of a quantity of stagnating water equal to or higher than one volume of water contained in the circuit and activating the said anti-stagnation system
- by calculating the time elapsed from the last use of water in the dental unit through an automatic program and/or;
- automatically at the start of the apparatus and/or;
- at pre-determined time intervals and/or;
- through a manual command by the operator.

2. Dental unit (1) with an anti-stagnation system according to claim 1, wherein said valve (16) is placed along water circuit (13) immediately downstream the last branch of the same circuit (13) towards the instruments (14) of instrument table (4).

3. Dental unit (1) with an anti-stagnation system according to claim 1 or 2, further comprising a branch (20) supplying potable water to assistant table (5); said branch (20) comprising an own anti-stagnation valve (26), placed downstream the branching of branch (20) towards instruments (21), which can be open or closed through the same automatic program (30) and/or manual control (31); when open, it directs the water contained in said branch (20) to an anti-stagnation discharge line (27).

4. Dental unit (1) with an anti-stagnation system according to claim 1 - 3, wherein said valve (16) is obtained as third way inside one of instrument supply valves (214 and/or 221).

5. Dental unit (1) with an anti-stagnation system according to claim 1 - 4, further comprising a disinfection system of water circuit (13) wherein automatic program (30), during the disinfection cycle of circuit (13) performs at least a step of opening valve (16 and optionally 26) extending the supply of disinfectant to the anti-stagnation discharge branches (17) and optionally (27).

6. Dental unit (1) with an anti-stagnation system according to claim 1-5, wherein the activation of an anti-stagnation valve (16 and/or 26) through automatic program (30) and/or manual control (31) also activates, in the branch of circuit (13) supplying water-to-cup spout (11), the contemporary or sequential opening of the (not shown) valve supplying water to water-to-cup spout (11).

7. Method for working anti-stagnation system (1) according to claims 1-6, wherein the method comprises the steps of
- Calculating by an automatic program (30) the interval of time elapsed since the last use of an instrument (14, 21) supplying water supported on instrument table (4), or optionally on assistant table (5), and of water-to-cup spout (11);
- Determining programmable opening times and programmable intervals through the automatic program (30);
- Activating the anti-stagnation valve (16) and optionally a valve (26)and optionally the water-to-cup spout (11) for the said programmable opening time and at the said programmable intervals by the automatic program (30).

8. Method for working the anti-stagnation system (1) according to claim 7, wherein the activation of anti-stagnation valve (16) and optionally of valve (26) and optionally of water-to-cup spout occurs, through an automatic program (30), at each switching on of the dental unit (1) itself.

9. Method for working anti-stagnation system (1) according to claim 7 or 8, wherein the activation of anti-stagnation valve (16) and optionally of valve (26), and optionally of water-to-cup spout (11), occurs, through an automatic program (30), each time that water in circuit (13) itself crosses a pre-set limit of microbial count, detected through means internal to the dental unit itself.

10. Method for the working of the anti-stagnation system (1) according to claim 7-9, wherein the activation of anti-stagnation valve (16) and optionally of valve (26) occurs through a manual control (31) activated by a human operator.

## Patentansprüche

1. Zahnärztliche Behandlungseinheit (1) mit einem Wasserkreislauf (13) zum Versorgen von Trinkwasser von der Eingangsstelle (12) zu den auf dem Instrumententisch (4) gelagerten Instrumenten (14), umfassend ein Antistagnationssystem
wobei das Anti-Stagnationssystem ein entlang des Wasserkreislaufs (13) angeordnetes Ventil (16) umfasst, das die Instrumente (14) auf dem Instrumententisch (4) versorgt, wobei das Ventil gemäß einem automatischen Programm (30) und/oder einer manuellen Bedienung (31) geöffnet oder geschlossen werden kann und, wenn es geöffnet ist, das Wasser aus dem Kreislauf (13) zu einer Antistagnationabflussleitung (17a oder 17b) leitet, die zum Abfluss (18) der zahnärztlichen Behandlungseinheit führt,
wobei das besagte Antistagnationssystem das automatische Programm (30), die manuelle Bedienung (31) und zwei alternative Abflussleitungen aufweist, umfassend:
- eine Leitung (17b) in Richtung des Abflusses (18) der zahnärztlichen Behandlungseinheit und
- eine Leitung (17a) des Speibeckens in Richtung des Abflusses (18) der zahnärztlichen Behandlungseinheit,
- das besagte automatische Programm (30) so konfiguriert ist, dass es programmierbare Öffnungszeiten des Antistagnationsventils (16) bestimmt, um mindestens den Abfluss einer Menge an stagnierendem Wasser zu gewährleisten, die gleich oder größer ist als ein im Kreislauf enthaltenes Wasservolumen, und das besagte Antistagnationssystem aktiviert
- durch Berechnung der Zeit, die seit der letzten Verwendung von Wasser in der zahnärztlichen Behandlungseinheit durch ein automatisches Programm verstrichen ist und/oder;
- automatisch beim Start der Vorrichtung und/oder;
- in vorbestimmten Zeitintervallen und/oder,
- durch eine manuelle Bedienung durch den Benutzer.

2. Zahnärztliche Behandlungseinheit (1) mit einem Antistagnationssystem nach Anspruch 1, wobei das Ventil (16) entlang des Wasserkreislaufs (13) unmittelbar stromabwärts der letzten Abzweigung desselben Kreislaufs (13) in Richtung der Instrumente (14) des Instrumententisches (4) angeordnet ist.

3. Zahnärztliche Behandlungseinheit (1) mit einem Antistagnationssystem nach Anspruch 1 oder 2, die außerdem eine Abzweigung (20) umfasst, die den Assistenztisch (5) mit Trinkwasser versorgt, wobei die Abzweigung (20) ein eigenes Antistagnationsventil (26) umfasst, das stromabwärts der Verzweigung der Abzweigung (20) in Richtung der Instrumente (21) angeordnet ist und das durch dasselbe automatische Programm (30) und/oder die manuelle Bedienung (31) geöffnet oder geschlossen werden kann und das, wenn es geöffnet ist, das in der Abzweigung (20) enthaltene Wasser zu einer Antistagnationsabflussleitung (27) leitet.

4. Zahnärztliche Behandlungseinheit (1) mit einem Antistagnationssystem nach Anspruch 1 bis 3, wobei das Ventil (16) als dritter Weg innerhalb eines der Instrumentenversorgungsventile (214 und/oder 221) erhalten wird.

5. Zahnärztliche Behandlungseinheit (1) mit einem Antistagnationssystem nach Anspruch 1 bis 4, ferner umfassend ein Desinfektionssystem des Wasserkreislaufs (13), wobei das automatische Programm (30) während des Desinfektionszyklus des Kreislaufs (13) mindestens einen Schritt des Öffnens des Ventils (16 und optional 26) durchführt, der die Zufuhr von Desinfektionsmittel bis zu den Antistagnationsabflusszweigen (17) und optional (27) erweitert.

6. Zahnärztliche Behandlungseinheit (1) mit einem Antistagnationssystem nach Anspruch 1-5, wobei die Aktivierung eines Antistagnationsventils (16 und/oder 26) durch ein automatisches Programm (30) und/oder eine manuelle Bedienung (31) auch die gleichzeitige oder aufeinanderfolgende Öffnung des den Wasser-zum-Becher-Auslauf (11) versorgenden Ventils (nicht dargestellt) in der den Wasser-zum-Becher-Auslauf (11) versorgenden Zweigleitung (13) aktiviert.

7. Verfahren zum Betreiben eines Antistagnationssystems (1) nach einem der Ansprüche 1 bis 6, wobei das Verfahren die folgenden Schritte umfasst
- Berechnen durch ein automatisches Programm (30) des Zeitintervalls seit der letzten Verwendung eines auf dem Instrumententisch (4) oder optional auf dem Assistenztisch (5) abgestellten wasserführenden Instruments (14, 21) und des Wasser-zum-Becher-Auslaufs (11);
- Bestimmen von programmierbaren Öffnungszeiten und programmierbaren Intervallen durch das automatische Programm (30);
- Aktivieren des Antistagnationsventils (16) und optional eines Ventils (26) und optional des Wasser-zum-Becher-Auslaufs (11) für die besagte programmierbare Öffnungszeit und mit den besagten programmierbaren Intervallen durch das automatische Programm (30).

8. Verfahren zum Betreiben des Antistagnationssystems (1) nach Anspruch 7, wobei die Aktivierung des Antistagnationsventils (16) und optional des Ventils (26) und optional des Wasser-zum-Becher-Auslaufs durch ein automatisches Programm (30) bei jedem Einschalten der zahnärztlichen Behandlungseinheit (1) selbst erfolgt.

9. Verfahren zum Betreiben eines Antistagnationssystems (1) nach Anspruch 7 oder 8, bei dem die Aktivierung des Antistagnationsventils (16) und optional des Ventils (26) und optional des Wasser-zum-Becher-Auslaufs (11) durch ein automatisches Programm (30) jedes Mal erfolgt, wenn das Wasser im Kreislauf (13) selbst einen voreingestellten und durch innerhalb der zahnärztlichen Einheit selbst befindliche Mittel erfassten Grenzwert der Mikrobenanzahl überschreitet.

10. Verfahren zum Betreiben des Antistagnationssystems (1) nach Anspruch 7 bis 9, wobei die Aktivierung des Antistagnationsventils (16) und optional des Ventils (26) durch eine manuelle Bedienung (31) erfolgt, die durch einen menschlichen Benutzer aktiviert wird.

## Revendications

1. Unité dentaire (1) comprenant un circuit d'eau (13) alimentant en eau potable depuis le point d'entrée (12) jusqu'aux instruments (14) supportés par le plateau (4) à instruments, comprenant un système anti-stagnation où
ledit système anti-stagnation comprend une vanne (16) placée le long du circuit d'eau (13) alimentant les instruments (14) sur le plateau (4) à instruments; ladite vanne étant ouverte ou fermée, selon un programme automatique (30) et/ou une commande manuelle (31); lorsqu'elle est ouverte, elle achemine l'eau du circuit (13) vers une ligne d'évacuation anti-stagnation (17a ou 17b) conduisant au conduit d'évacuation (18) de l'unité dentaire,
ledit système anti-stagnation étant muni d'un programme automatique (30), de la commande manuelle (31) et de deux lignes d'évacuation alternatives consistant en:
- une ligne (17b) vers le conduit d'évacuation (18) de l'unité dentaire et
- une ligne (17a) du crachoir vers le conduit d'évacuation (18) de l'unité dentaire,
ledit programme automatique (30) étant configuré pour déterminer des périodes d'ouverture programmables de la valve anti-stagnation (16) pour garantir, au minimum, l'évacuation d'une quantité d'eau stagnante égale ou supérieure à un volume d'eau contenu dans le circuit et activant ledit système anti-stagnation
- en calculant le temps passé depuis la dernière utilisation d'eau dans l'unité dentaire au moyen d'un programme automatique et/ou;
- automatiquement au démarrage de l'appareil et/ou;
- à des intervalles de temps prédéterminés et/ou;
- par une commande manuelle de l'opérateur.

2. Unité dentaire (1) avec un système anti-stagnation selon la revendication 1, où ladite vanne (16) est placée le long du circuit d'eau (13) immédiatement en aval du dernier embranchement du même circuit (13) vers les instruments (14) du plateau (4) à instruments.

3. Unité dentaire (1) avec un système anti-stagnation selon la revendication 1 ou 2, comprenant en outre un embranchement (20) fournissant de l'eau potable au plateau (5) de l'assistant; ledit embranchement (20) comprenant une propre vanne anti-stagnation (26), placée en aval de la division de l'embranchement (20) vers les instruments (21), qui peut être ouverte ou fermée par le même programme automatique (30) et/ou la commande manuelle (31); lorsqu'elle est ouverte, elle achemine l'eau contenue dans ledit embranchement (20) vers une ligne d'évacuation anti-stagnation (27).

4. Unité dentaire (1) avec un système anti-stagnation selon la revendication 1-3, où ladite vanne (16) est obtenue comme troisième voie à l'intérieur d'une des vannes d'alimentation (214 et/ou 221) des instruments.

5. Unité dentaire (1) avec un système anti-stagnation selon les revendications 1-4, comprenant en outre un système de désinfection du circuit d'eau (13) où le programme automatique (30), pendant le cycle de désinfection du circuit (13), exécute au moins une étape d'ouverture de la vanne (16 et éventuellement 26) étendant l'alimentation en désinfectant aux embranchements d'évacuation anti-stagnation (17) et éventuellement (27).

6. Unité dentaire (1) avec un système anti-stagnation selon les revendications 1-5, où l'activation d'une valve anti-stagnation (16 et/ou 26) par le programme automatique (30) et/ou une commande manuelle (31) active également, dans l'embranchement du circuit (13) fournissant l'eau au bec verseur (11), l'ouverture simultanée ou séquentielle de la valve (non représentée) fournissant l'eau au bec verseur (11).

7. Méthode de fonctionnement du système anti-stagnation (1) selon les revendications 1-6, où la méthode comprend les étapes suivantes
- Calculer par un programme automatique (30) l'intervalle de temps passé depuis la dernière utilisation d'un instrument (14, 21) fournissant de l'eau, supporté par le plateau (4) à instruments ou éventuellement par un plateau (5) de l'assistant, et d'un bec verseur (11);
- Déterminer des périodes d'ouverture programmables et des intervalles programmables par le programme automatique (30);
- Activer la valve anti-stagnation (16) et éventuellement une valve (26) et éventuellement le bec verseur (11) pour ladite période d'ouverture programmable et auxdits intervalles programmables par le programme automatique (30).

8. Méthode de fonctionnement du système anti-stagnation (1) selon la revendication 7, où l'activation de la valve anti-stagnation (16) et éventuellement de la valve (26) et éventuellement du bec verseur se produit, par le biais d'un programme automatique (30), à chaque mise en marche de l'unité dentaire (1) elle-même.

9. Méthode de fonctionnement du système anti-stagnation (1) selon la revendication 7 ou 8, où l'activation de la vanne anti-stagnation (16) et éventuellement de la vanne (26), et éventuellement du bec verseur (11), se produit, par le biais d'un programme automatique (30), chaque fois que l'eau dans le circuit (13) lui-même franchit une limite prédéfinie du dénombrement microbien, détecté par des moyens internes à l'unité dentaire elle-même.

10. Méthode pour le fonctionnement du système anti-stagnation (1) selon la revendication 7-9, où l'activation de la vanne anti-stagnation (16) et éventuellement de la vanne (26) se produit par une commande manuelle (31) activée par un opérateur humain.
